(19) Europäisches Patentamt
European Patent Office
Office européen des brevets

(11) Veröffentlichungsnummer: **0 231 845 B1**

(12) **EUROPÄISCHE PATENTSCHRIFT**

(45) Veröffentlichungstag der Patentschrift: **17.06.92**

(51) Int. Cl.⁵: **C07D 501/06**, C07D 499/12, C07D 277/74

(21) Anmeldenummer: **87101012.0**

(22) Anmeldetag: **24.01.87**

(54) **Verfahren zur Herstellung von Carbonsäureamiden.**

(30) Priorität: **07.02.86 CH 487/86**

(43) Veröffentlichungstag der Anmeldung:
**12.08.87 Patentblatt 87/33**

(45) Bekanntmachung des Hinweises auf die Patenterteilung:
**17.06.92 Patentblatt 92/25**

(84) Benannte Vertragsstaaten:
**AT BE CH DE ES FR GB GR IT LI LU NL SE**

(56) Entgegenhaltungen:
**EP-A- 0 037 380**
**EP-A- 0 115 770**
**EP-A- 0 187 209**
**WO-A-85/04659**

(73) Patentinhaber: **F. HOFFMANN-LA ROCHE AG**
**Postfach 3255**
**CH-4002 Basel(CH)**

(72) Erfinder: **Furlenmeier, André, Dr.**
**Wettsteinallee 119**
**CH-4058 Basel(CH)**
Erfinder: **Weiss, Urs**
**Wyhlenstrasse 20A**
**CH-4133 Pratteln(CH)**

(74) Vertreter: **Notegen, Eric-André et al**
**Grenzacherstrasse 124 Postfach 3255**
**CH-4002 Basel(CH)**

EP 0 231 845 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von Carbonsäureamiden durch Acylierung von Aminen mit 2-Benzothiazolylthioestern von Carbonsäuren, das dadurch gekennzeichnet ist, dass man das Amin in Form eines Säureadditionssalzes zur Umsetzung bringt.

Die Acylierung von Aminen mit 2-Benzothiazolylthioestern ist eine an sich bekannte Methode zur Herstellung von Carbonsäureamiden. Bei dieser Methode wird das Amin in Form der freien Base zur Umsetzung gebracht, wobei man häufig noch eine zusätzliche organische Base, in der Regel ein tertiäres Amin, zum Reaktionsgemisch gibt, um den Reaktionsablauf zu beschleunigen. In Fällen in denen man ein Säureadditionssalz eines Amins als Ausgangsstoff verwendet hat, war man bisher der Meinung, dass man mindestens ein Aequivalent einer organischen Base, in der Regel eines tertiären Amins, dem Reaktionsgemisch beifügen müsse, um das Säureadditionssalz in die reaktive Form, d.h. in die freie Base überzuführen.

Es wurde nun überraschenderweise gefunden, dass Säureadditionssalze von Aminen direkt mit 2-Benzothiazolylthioestern von Carbonsäuren umgesetzt werden können, d.h. dass es nicht notwendig ist, das Säureadditionssalz des Amins vorgängig mit einer organischen Base zu neutralisieren, um die reaktive Form, nämlich das freie Amin, freizusetzen. Wie die weiter unten folgenden Beispiele belegen, ist das erfindungsgemässe Verfahren nicht auf bestimmte 2-Benzothiazolylthioester oder auf bestimmte Säureadditionssalze von Aminen beschränkt. Es ist vielmehr allgemein anwendbar.

Das erfindungsgemässe Verfahren ist insbesondere dann von grossem Vorteil, wenn das Amin in Form der freien Base nicht besonders stabil ist, was insbesondere bei 7-Amino-cephalosporinderivaten häufig der Fall ist. Mit dem erfindungsgemässen Verfahren können 7-Acylamino-cephalosporinderivate in guter Ausbeute und in grosser Reinheit hergestellt werden.

Gegenstand der vorliegenden Erfindung sind demnach ein Verfahren zur Acylierung von Aminen mit 2-Benzothiazolylthioestern von Carbonsäuren, das dadurch gekennzeichnet ist, dass man das Amin in Form eines Säureadditionssalzes zur Umsetzung bringt, sowie die Anwendung dieses Verfahrens zur Herstellung von Carbonsäureamiden, insbesondere von antimikrobiell wirksamen 7-Acylamino-cephalosporinderivaten.

Wie bereits erwähnt, ist das erfindungsgemässe Verfahren jedoch nicht auf bestimmte Amine beschränkt. Es können beispielsweise auch 6-Amino-penicillinderivate oder auch einfache aliphatische, alicyclische oder aromatische Amine, wie Anilin, erfolgreich acyliert werden.

Das erfindungsgemässe Verfahren kann in jedem inerten organischen Lösungsmittel durchgeführt werden, in welchem die Ausgangsstoffe mindestens teilweise löslich sind. Als Lösungsmittel kommen beispielsweise die nachfolgend aufgeführten in Frage: Halogenierte niedere Kohlenwasserstoffe, wie Methylenchlorid, Chloroform und Tetrachlorkohlenstoff, niedere N,N-Dialkylfettsäureamide, wie N,N-Dimethylacetamid und N,N-Dimethylformamid, niedere Alkohole, wie Methanol und Aethanol, niedere Dialkyläther, wie Diäthyläther, Dimethyläther und t-Butylmethyläther, niedere cyclische Aether, wie Tetrahydrofuran und Dioxan, niedere Mono- und Dialkyläther von Alkandiolen, wie Aethylenglykoldiäthyläther und Aethylenglykolmonomethyläther,niedere Dialkylketone, wie Aceton und Methyläthylketon, Dimethylsulfoxid und Mischungen der vorerwähnten Lösungsmittel.

Bevorzugte Lösungsmittel sind die halogenierten niederen Kohlenwasserstoffe, insbesondere die chlorierten niederen Kohlenwasserstoffe, die niederen Alkohole und die niederen N,N-Dialkylfettsäureamide. Ein ganz besonders bevorzugtes Lösungsmittel ist Methylenchlorid.

Das erfindungsgemässe Verfahren kann in einem weiten Temperaturbereich durchgeführt werden. Es kann beispielsweise in einem Bereich von etwa 0 °C bis etwa 60 °C durchgeführt werden. Die Umsetzung wird vorzugsweise in einem Temperaturbereich von etwa 15 °C bis etwa 30 °C durchgeführt, wobei man in einer ganz besonders bevorzugten Ausführungsform bei Raumtemperatur arbeitet.

Als Säureadditionssalze kommen sowohl Salze mit anorganischen als auch Salze mit organischen Säuren in Betracht. Vorzugsweise verwendet man Säureadditionssalze mit aromatischen oder aliphatischen Sulfonsäuren oder Mineralsäuren. In einer besonders bevorzugten Ausführungsform verwendet man Säureadditionssalze mit p-Toluolsulfonsäure oder Salzsäure.

Als Amine verwendet man vorzugsweise eine Verbindung der allgemeinen Formel

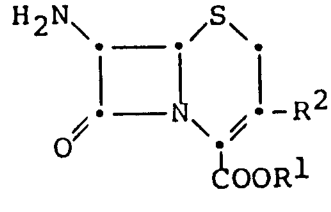

I

worin $R^1$ Wasserstoff oder eine für die Blockierung der 4-Carboxygruppe von Cephalosporinen übliche Gruppe und $R^2$ Wasserstoff oder eine für die 3-Stellung von Cephalosporinen übliche Gruppe bedeuten.

In einer besonders bevorzugten Ausführungsform verwendet man Amine der obigen Formel I worin $R^1$ Wasserstoff oder die Gruppe -A-OCOR, A niederes Alkyliden, R niederes Alkyl oder niederes Alkoxy, $R^2$ Wasserstoff, Halogen, niederes Alkyl, niederes Alkenyl, niederes Alkanoyloxyalkyl, niederes Azidoalkyl, niederes Carbamoyloxyalkyl oder die Gruppe $-CH_2-S-Q$, $-CH=CH-S-Q$ oder $-CH_2-Q'$, Q eine über ein Kohlenstoffatom gebundene heterocyclische Gruppe und Q' eine über ein Stickstoffatom gebundene, N-haltige heterocyclische Gruppe bedeuten, wobei es sich bei den heterocyclischen Gruppen um bei Cephalosporinen übliche Gruppen handelt.

Als 2-Benzothiazolylthioester von Carbonsäuren verwendet man vorzugsweise eine Verbindung der allgemeinen Formel

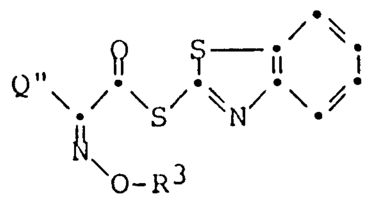

II

worin $R^3$ niederes Alkyl, niederes Alkenyl, niederes Alkanoyl oder die Gruppe -A'-COOR', A' niederes Alkylen, R' eine in der Cephalosporinchemie übliche Carboxyschutzgruppe und Q" eine über ein Kohlenstoffatom gebundene heteroaromatische Gruppe bedeuten, wobei es sich bei den heteroaromatischen Gruppen um bei 7-Oxyiminoacetylamino-Cephalosporinen übliche Gruppen handelt.

In einer besonders bevorzugten Ausführungsform verwendet man eine Verbindung der obigen Formel II, worin $R^3$ Methyl oder die Gruppe -A'-COOR', A' Methylen oder 2,2-Propylen, R' t-Butyl und Q" 2-Amino-4-thiazolyl oder 2-Furanyl bedeuten.

Bei der Umsetzung eines Säureadditionssalzes eines Amins der Formel I mit einer Verbindung der Formel II erhält man zunächst eine Verbindung der allgemeinen Formel

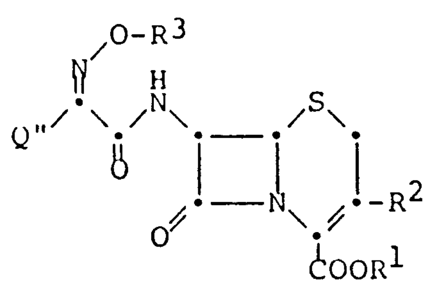

III

worin $R^1$, $R^2$, $R^3$ und Q" obige Bedeutung besitzen, oder, sofern eine zur Salzbildung befähigte Aminogruppe vorhanden ist, das entsprechende Säureadditionssalz davon. Bei diesen Verbindungen handelt es sich um bekannte Verbindungen, welche antimikrobielle Eigenschaften besitzen oder welche nach bekannten Methoden in antimikrobiell wirksame Verbindungen übergeführt werden können.

Bei den antimikrobiell wirksamen 7-Acylamino-cephalosporinderivaten, welche in einer bevorzugten Ausführungsform erfindungsgemäss hergestellt werden können, handelt es sich um die Verbindungen der allgemeinen Formel

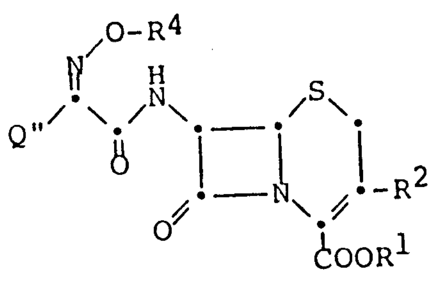

IV

worin $R^4$ Wasserstoff, niederes Alkyl, niederes Alkenyl oder die Gruppe -A'-COOH bedeutet, und $R^1$, $R^2$, A' und Q'' obige Bedeutung besitzen,
und die pharmazeutisch annehmbaren Salze davon.

In einer ganz besonders bevorzugten Ausführungsform verwendet man als Amin Methylen (6R,7R)-7-amino-3-[(5-methyl-2H-tetrazol-2-yl)methyl]-8-oxo -5-thial-1-azabicyclo[4.2.0]oct-2-en-carboxylat-pivalat und als Benzthiazolylthioester den (Z)-2-(2-Amino-4-thiazolyl)-2-(methoxyimino)essigsäure -2-benzthiazolylthioester, wobei man ein Säureadditionssalz von Methylen (6R,7R)-7-[(Z)-2-(2-Amino-4-thiazolyl) -2-(methoxyimino)acetamido]-3-[(5-methyl-2H-tetrazol -2-yl)methyl]-8-oxo-5-thia-1-azabicyclo[4.2.0]oct-2 -en-carboxylat-pivalat erhält.

Im Rahmen der vorliegenden Erfindung bezeichnet der Ausdruck "nieder" Reste und Verbindungen mit bis zu 7, vorzugsweise bis zu 4 Kohlenstoffatomen. Der Ausdruck "Alkyl" für sich allein genommen oder in Kombinationen, wie Alkoxy, Alkylen, Alkyliden und Azidoalkyl, bezeichnet geradkettige oder verzweigte, gesättigte Kohlenwasserstoffreste, wie Methyl, Aethyl, Isopropyl und s-Butyl. Der Ausdruck "Alkenyl" bezeichnet geradkettige und verzweigte, ungesättigte Kohlenwasserstoffreste, wie Allyl. Der Ausdruck "Alkanoyl" bezeichnet geradkettige oder verzweigte Fettsäurereste, wie Acetyl.

Der Ausdruck "heteroaromatische Gruppe" bezeichnet vorzugsweise monocyclische heteroaromatische Gruppen, welche als Heteroatome vorzugsweise ein Sauerstoff- oder Schwefelatom und/oder 1-4 Stickstoffatome enthalten. Sie sind vorzugsweise 5- oder 6-gliedrig. Sie können gegebenenfalls substituiert sein, wobei als Substituenten beispielsweise Amino- und niedere Alkylgruppen in Frage kommen. 2-Furanyl, 2-Amino-4-thiazolyl und 5-Methyl-1,3,4-thiadiazol-2-yl sind Beispiele für heteroaromatische Gruppen.

Der Ausdruck "heterocyclische Gruppe" bezeichnet vorzugsweise monocyclische, insbesondere 5- und 6-gliedrige, partiell ungesättigte oder aromatische heterocyclische Gruppen, welche als Heteroatome vorzugsweise ein Sauerstoff- oder Schwefelatom und/oder 1-4 Stickstoffatome enthalten, oder bicyclische, insbesondere 8- bis 10-gliedrige, partiell ungesättigte oder aromatische heterocyclische Gruppen, welche als Heteroatome vorzugsweise ein Sauerstoff- oder Schwefelatom und/oder 1-5 Stickstoffatome enthalten. Diese Gruppen sind vorzugsweise unsubstituiert oder durch Hydroxy, Oxo, niederes Alkyl und/oder niederes Oxoalkyl substituiert.

Der Ausdruck "N-haltige heterocyclische Gruppe" bezeichnet vorzugsweise gesättigte, partiell ungesättigte und aromatische heterocyclische Gruppen, welche bis zu 4 Stickstoffatome als Heteroatome enthalten. Sie sind vorzugsweise 5- oder 6-gliedrig und können noch durch einen 5- oder 6-gliedrigen Cycloalkan- oder Benzolring anneliert sein. Sie sind vorzugsweise unsubstituiert oder durch niederes Alkyl oder Carbamoyl substituiert. Das Stickstoffatom, über welches die N-haltige heterocyclische Gruppe gebunden ist, kann auch quaternär substituiert sein. 5-Methyl-2-tetrazolyl und Pyridinium-1-yl sind Beispiele für N-haltige heterocyclische Gruppen.

Die nachfolgenden Beispiele dienen der näheren Erläuterung der vorliegenden Erfindung.

Beispiel 1

a) Man suspendiert 8,77 g (6R,7R)-7-Amino-3-methyl-8-oxo-5-thia-1-azabicyclo[4.2.0]oct -2-en-2-carbonsäure in 60 ml Aceton, versetzt innerhalb von 15 Minuten bei 20-25° unter Rühren mit 6,3 g 1,8-Diazabicyclo[5.4.0]undec-7-en (DBU), gibt zur entstandenen Lösung bei 15° 10,6 g Pivaloyloxymethyljodid und rührt 15 Minuten ohne Kühlung. Dann werden 250 ml n-Butylacetat dazugegeben, worauf man vom auskristallisierten DBU-Hydrojodid abnutscht und mit 50 ml n-Butylacetat nachwäscht. Das gelbe Filtrat wird zweimal mit 125 ml ges. Kochsalzlösung gewaschen und durch ein Faltenfilter filtriert. Am Wasserstrahlvakuum werden bei 35° 100 ml Lösungsmittel abdestilliert. Die Lösung wird nochmals filtriert und unter Rühren mit 8,4 g p-Toluolsulfonsäure$\bullet H_2O$ versetzt, wobei das Produkt auskristallisiert. Nach Rühren während 30 Minuten wird abgenutscht, mit n-Butylacetat und n-Hexan gewaschen und über Nacht im Wasserstrahlvakuum bei 35° getrocknet. Man erhält 18,5 g (92,36%) Methylen-(6R,7R)-7-amino-3-methyl-8-oxo-5-thia -1-azabicyclo[4.2.0]oct-2-en-2-carboxylat-pivalat p-Toluolsulfonat als weisse Kristalle ($C_{14}H_{20}N_2O_5S\bullet C_7H_8SO_3$; MG: 500, 581).

| Mikroanalyse: | | |
|---|---|---|
| | Ber. | Gef. |
| C | 50,39 | 50,45 |
| H | 5,64 | 5,78 |
| N | 5,60 | 5.44 |

b) 10,02 g Methylen (6R,7R)-7-amino-3-methyl-8-oxo-5-thia -1-azabicyclo[4.2.0]oct-2-en-2-carboxylat-pivalat p-Toluolsulfonat werden in 100 ml Methylenchlorid gelöst, worauf man mit 7,0 g S-(2-Benzothiazolyl)-2-amino-4-thiazol-thioglyoxylat (Z)-O-methyloxim versetzt, während 1,5 Stunden bei 20-25° rührt, die entstandene Lösung zweimal mit je 50 ml 5-proz. Natriumacetatlösung und einmal mit 50 ml Wasser wäscht, durch ein Faltenfilter filtriert und im Wasserstrahlvakuum bei 30° eindampft. Der Rückstand wird in 100 ml Isopropanol unter Zusatz von 4,5 ml 5,2N-Salzsäure in Isopropanol gelöst. Man rührt während 15 Minuten bei 20-25°, wobei massive Kristallisation eintritt. Man tropft innerhalb von 30 Minuten 100 ml n-Hexan unter Rühren dazu, filtriert vom auskristallisierten Material ab, wäscht mit Isopropanol/n-Hexan (1:1) und n-Hexan und trocknet über Nacht im Wasserstrahlvakuum bei 40°. Man erhält 10,25 g (93,5%) Methylen-(6R,7R)-7-[(Z)-2-(2-amino-4-thiazolyl) -2-(methoxyimino)acetamido]-3-methyl-8-oxo-5-thia -1-azabicyclo[4.2.0]oct-2-en-2-carboxylat-pivalat Hydrochlorid als farblose Kristalle. Dieses Material wird in 50 ml Aethanol bei 40° gelöst. Man tropft dann 30 ml n-Hexan dazu und lässt während 15 Minuten unter Rühren kristallisieren. Nach Zutropfen von weiteren 20 ml n-Hexan wird abgenutscht, mit Aethanol/n-Hexan (1:1) und n-Hexan gewaschen und im Wasserstrahlvakuum bei 40° getrocknet. Man erhält 8,9 g (86,8%) des gewünschten Produktes. ($C_{20}H_{25}N_5O_7S_2 \cdot HCl$; MG: 548, 029).

| Mikroanalyse: | | |
|---|---|---|
| | Ber. | Gef. |
| C | 43,83 | 43.85 |
| H | 4,78 | 4,86 |
| N | 12.78 | 12,83 |

Beispiel 2

a) 22,3 g (6R,7R)-7-Amino-3-acetoxymethyl-8-oxo-5-thia -1-azabicyclo[4.2.0]oct-2-en-2-carbonsäure werden in 120 ml Aceton suspendiert, worauf man innerhalb von 15 Minuten unter Rühren bei 20-25° mit 12,6 g 1,8-Diazabicyclo[5.4.0]undec-7-en (DBU) versetzt. Zur entstandenen Lösung werden bei 15° 21,2 g Pivaloyloxymethyljodid gegeben, und es wird während 15 Minuten ohne Kühlung gerührt. Nun werden 500 ml n-Butylacetat zugegeben, worauf man vom auskristallisierten DBU-Hydrojodid abnutscht und mit 100 ml n-Butylacetat wäscht. Das gelbe Filtrat wird zweimal mit je 250 ml ges. Kochsalzlösung gewaschen und durch ein Faltenfilter filtriert. Im Wasserstrahlvakuum werden bei 35° 200 ml Lösungsmittel abdestilliert. Die erhaltene Lösung wird nochmals filtriert und unter Rühren mit 16,8 g p-Toluolsulfonsäure$\cdot H_2O$ versetzt, wobei das Produkt auskristallisiert. Nach 30 Minuten Rühren wird abgenutscht, mit n-Butylacetat und n-Hexan gewaschen und über Nacht im Wasserstrahlvakuum bei 35° getrocknet. Man erhält 38,7 g (86,6%) Methylen-(6R,7R)-3-(acetoxymethyl)-7-amino-8-oxo-5-thia -1-azabicyclo[4.2.0]oct-2-en-2-carboxylat-pivalat p-Toluolsulfonat als farblose Kristalle ($C_{16}H_{22}N_2O_7S \cdot C_7H_8SO_3$; MG: 558, 617).

| Mikroanalyse: | | |
|---|---|---|
| | Ber. | Gef. |
| C | 49,45 | 49,52 |
| H | 5,41 | 5,55 |
| N | 5,01 | 4,95 |

b) 5,58 g Methylen-(6R,7R)-3-(acetoxymethyl)-7-amin-8-oxo-5-thia -1-azabicyclo[4.2.0]oct-2-en-2-carboxylat-pivalat p-Toluolsulfonat werden in 60 ml Methylenchlorid zusammen mit 3,5 g S-(2-Benzothiazolyl)-2-amino-4-thiazol-thioglyoxylat (Z)-O-methyloxim während 2 Stunden bei 20-25° gerührt. Die entstandene Lösung wird zweimal mit 30 ml 5-proz. Natriumacetatlösung und einmal mit 30 ml Wasser gewaschen, durch ein Faltenfilter filtriert und im Wasserstrahlvakuum bei 40° eingedampft. Der Rückstand wird in 50 ml Essigester unter Zusatz von 2,5 ml 5,2N-Salzsäure in Isopropanol gelöst. Es wird 100 ml Aether dazugegeben, wobei ein harziges Material ausfällt. Die überstehende Flüssigkeit wird abdekantiert, und das Harz wird in 50 ml Essigester gelöst und durch langsame Zugabe von Aether kristallisiert. Es wird abgenutscht, mit Aether und Petroläther gewaschen und über Nacht im Wasserstrahlvakuum bei 30° getrocknet. Man erhält 3,7 g (61%) Methylen 3-(acetoxymethyl)-(6R,7R)-7-[(Z)-2-(2-amino-4-thiazolyl) -2-(methoxyimino)acetamido]-8-oxo-5-thia -1-azabicyclo[4.2.0]oct-2-en-2-carboxylat Hydrochlorid als beige Kristalle ($C_{22}H_{27}N_5O_9S_2 \cdot HCl$; MG; 606, 065).

| Mikroanalyse: | | |
|---|---|---|
| | Ber. | Gef. ($H_2O$-frei) |
| C | 43.60 | 43,81 |
| H | 4,66 | 4,72 |
| N | 11,56 | 11,58 |

Beispiel 3

a) 11,5 g (6R,7R)-7-Amino-3-azidomethyl-8-oxo-5-thia -1-azabicyclo[4.2.0]oct-2-en-2-carbonsäure werden in 250 ml Methylenchlorid suspendiert. Man versetzt bei 20-25° unter Rühren mit 6,5 g 1,8-Diazabicyclo-[5.4.0]undec-7-en (DBU). Nach 15 Minuten Rühren gibt man zur dunklen Suspension 10,9 g Pivaloyloxymethyljodid und rührt während 20 Minuten bei 20-25°. Nun werden 200 ml Wasser zugegeben, und die Emulsion wird durch ein Filtrierhilfsmittel filtriert. Die organische Phase wird abgetrennt, zweimal mit je 100 ml Wasser gewaschen und mit 250 ml n-Butylacetat versetzt, worauf man am Wasserstrahlvakuum bei 25° 250 ml Lösungsmittel abdestilliert. Die Lösung wird durch ein Faltenfilter filtriert, und das Filtrat wird unter Rühren mit 7,6 g p-Toluolsulfonsäure$\cdot H_2O$ versetzt. Nach 30 Minuten Rühren wird vom auskristallisierten Material abgenutscht, mit n-Butylacetat und tiefs. Petroläther gewaschen und über Nacht am Wasserstrahlvakuum bei 35° getrocknet. Man erhält 11,5 g (53,10%) Methylen-(6R,7R)-7-amino-3-(azidomethyl)-8-oxo-5-thia -1-azabicyclo[4.2.0]oct-2-en-2-carboxylat-pivalat p-Toluolsulfonat als beige Kristalle ($C_{14}H_{19}N_5O_5S \cdot C_7H_8SO_3$; MG: 541, 594).

| Mikroanalyse: | | |
|---|---|---|
| | Ber. | Gef. |
| C | 46,57 | 47,02 |
| H | 5,03 | 5,18 |
| N | 12,93 | 12,49 |

b) 5,42 g Methylen-(6R,7R)-7-amino-3-(azidomethyl)-8-oxo-5-thia -1-azabicyclo[4.2.0]oct-2-en-2-carboxylat-pivalat p-Toluolsulfonat werden in 50 ml Methylenchlorid suspendiert, worauf man mit 3,5 g S-(2-Benzothiazolyl)-2-amino-4-thiazol-thioglyoxylat (Z)-O-methyloxim versetzt und 2 Stunden bei 20-25° rührt. Die entstandene Lösung wird zweimal mit je 50 ml 5-proz. Natriumacetatlösung und einmal mit 50 ml Wasser gewaschen, durch ein Faltenfilter filtriert und im Wasserstrahlvakuum bei 30° eingedampft. Der Rückstand wird in 50 ml Essigester unter Zusatz von 2,5 ml 5,2N-Salzsäure in Isopropanol gelöst. Unter Rühren werden 150 ml Aether zugegeben, worauf man vom amorph ausgefallenen Material abnutscht. Eine Lösung des erhaltenen Materials in 50 ml Essigester wird unter Rühren auf 150 ml Aether gegossen. Das amorph ausgefallene Material wird abgenutscht, mit Aether und tiefs. Petroläther gewaschen und über Nacht im Hochvakuum bei 30° getrocknet. Man erhält 5,0 g (84,75%) Methylen (6R,7R)-7-[(Z)-2-(2-amino-4-thiazolyl) -2-(methoxyimino)acetamido]-3-(azidomethyl)-8-oxo-5-thia -1-azabicyclo[4.2.0]oct-2-en-2-carboxylat-pivalat Hydrochlorid als beigen amorphen Festkörper ($C_{20}H_{24}N_8O_7S_2 \cdot HCl$; MG: 589,042).

| Mikroanalyse: | | |
|---|---|---|
| | Ber. | Gef. |
| C | 40,78 | 40,84 |
| H | 4,28 | 4,35 |
| N | 19,02 | 18,74 |

Beispiel 4

a) 15,6 g 7-Amino-3-[[(5-methyl-1,3,4-thiadiazol-2-yl)thio]methyl] -8-oxo-5-thia-1-azabicyclo[4.2.0]oct-2-en-2-carbonsäure werden in 250 ml Methylenchlorid suspendiert, worauf man unter Rühren bei 20-25° mit 6.5 g 1,8-Diazabicyclo[5.4.0]undec-7-en (DBU) versetzt. Nach 15 Minuten Rühren werden 10,9 g Pivaloyloxymethyljodid dazugegeben. Man rührt 20 Minuten bei 20-25°, gibt 250 ml Wasser dazu und filtriert die Emulsion durch ein Filtrierhilfsmittel. Die organische Phase wird abgetrennt, mit 250 ml Wasser gewaschen und mit 500 ml n-Butylacetat versetzt, worauf man im Wasserstrahlvakuum bei 35° auf ein Volumen von 400 ml einengt, filtriert und das Filtrat mit 7,6 g p-Toluolsulfonsäure•$H_2O$ unter Rühren versetzt. Nach 30 Minuten Rühren wird das auskristallisierte Material abgenutscht, mit n-Butylacetat und tiefs. Petroläther gewaschen und über Nacht im Wasserstrahlvakuum bei 35° getrocknet. Das erhaltene Material (12,0 g beige Kristalle) wird in 100 ml Methylenchlorid gelöst. Man versetzt mit 250 ml Essigester und engt die Lösung im Wasserstrahlvakuum bei 30° auf ein Volumen von 150 ml ein. Das auskristallisierte Material wird abgenutscht, mit Essigester und tiefs. Petroläther gewaschen und über Nacht im Wasserstrahlvakuum bei 30° getrocknet. Man erhält 8,2 g (32,5%) Methylen (6R,7R)-7-amino-3-[[(5-methyl-1,3,4-thiadiazol -2-yl)thio]methyl]-8-oxo-5-thia-1-azabicyclo[4.2.0]oct-2-en-2-carboxylat p-Toluolsulfonat als weisse Kristalle ($C_{17}H_{22}N_4O_5S_3$•$C_7H_8SO_3$; MG: 630,764).

| Mikroanalyse: | | |
|---|---|---|
| | Ber. | Gef. |
| C | 45,70 | 45,79 |
| H | 4,79 | 4,83 |
| N | 8,88 | 8,74 |

b) 6,3 g Methylen (6R,7R)-7-amino-3-[[(5-methyl-1,3,4-thiadiazol -2-yl)thio]methyl]-8-oxo-5-thia-1-azabicyclo[4.2.0]oct -2-en-2-carboxylat-pivalat p-Toluolsulfonat werden in 60 ml Methylenchlorid gelöst. Man versetzt mit 3,5 g S-(2-Benzothiazolyl)-2-amino-4-thiazol-thioglyoxylat (Z)-O-methyloxim, rührt während 2 Stunden bei 20-25°, wäscht die Lösung zweimal mit je 50 ml 5-proz. Natriumacetatlösung und einmal mit 50 ml Wasser, filtriert durch ein Faltenfilter und dampft im Wasserstrahlvakuum bei 30° ein. Der Rückstand wird in 100 ml Essigester gelöst. Unter Rühren gibt man 2,5 ml 5,2N-Salzsäure in Isopropanol dazu, nutscht das ausgefallene Hydrochlorid ab, wäscht mit Essigester und tiefs. Petroläther und trocknet über Nacht im Wasserstrahlvakuum bei 30°. Das erhaltene Material (6,2 g) wird in 30 ml Aceton gelöst. Unter Rühren giesst man auf 150 ml Aether, nutscht das amorph ausgefallene Material ab, wäscht mit Aether und Petroläther und trocknet über Nacht im Hochvakuum bei 40°. Man erhält 5,0 g (73,7%) Methylen (6R,7R)-7-[(Z)-2-(2-amino-4-thiazolyl) -2-(methoxyimino)acetamido]-3-[[(5-methyl-1,3,4-thiadiazol -2-yl)thio]methyl]-8-oxo-5-thial-1-azabicyclo[4.2.0]oct -2-en-2-carboxylat-pivalat Hydrochlorid als beige-amorphen Festkörper ($C_{23}H_{27}N_7O_7S_4$• HCl; MG: 678,212).

| Mikroanalyse: | | |
|---|---|---|
| | Ber. | Gef. |
| C | 40,73 | 40,48 |
| H | 4,16 | 4,15 |
| N | 14,46 | 14,10 |

Beispiel 5

a) 12,15 g (6R,7R)-7-Amino-3-(5-methyl-2H-tetrazol-2-yl)-8-oxo-5-thia -1-azabicyclo[4.2.0]oct-2-en-2-carbonsäure werden in 60 ml Aceton suspendiert. Man versetzt innerhalb von 15 Minuten bei 20-25° unter Rühren mit 6,3 g 1,8-Diazabicyclo[5.4.0]undec-7-en (DBU), gibt zur entstandenen Lösung bei 15° 10,6 g Pivaloyloxymethyljodid und rührt 15 Minuten ohne Kühlung. Nun werden 250 ml n-Butylacetat dazugegeben. Es wird vom auskristallisierten DBU-Hydrojodid abgenutscht und mit 50 ml n-Butylacetat gewaschen. Das gelbe Filtrat wird zweimal mit je 125 ml ges. Kochsalzlösung gewaschen und durch ein Faltenfilter filtriert. Im Wasserstrahlvakuum werden bei 35° 100 ml Lösungsmittel abdestilliert. Die Lösung wird nochmals filtriert und unter Rühren mit 8,4 g p-Toluolsulfonsäure•$H_2O$ versetzt. Das auskristallisierte Material wird nach 30 Minuten Rühren abgenutscht, mit n-Butylacetat gewaschen. Man suspendiert das erhaltene Material in 200 ml n-Butylacetat und versetzt unter Rühren mit 20 ml 5,2N-Salzsäure in Isopropanol, wobei eine Lösung entsteht. Diese Lösung wird während 1 Stunde gerührt, wobei Kristallisation eintritt. Man nutscht das auskristallisierte Hydrochlorid ab, wäscht mit n-Butylacetat und n-Hexan und trocknet über Nacht im Wasserstrahlvakuum bei 35°. Man erhält 13,9 g (77,75%) Methylen (6R,7R)-7-amino-3-[5-methyl-2H-tetrazol-2-yl)methyl]-8-oxo -5-thia-1-azabicyclo[4.2.0]oct-2-en-2-carboxylat-pivalat Hydrochlorid als weisse Kristalle ($C_{16}H_{22}N_6O_5S$•HCl; MG: 446,91).

| Mikroanalyse: | | |
|---|---|---|
| | Ber. | Gef. |
| C | 43,00 | 43,05 |
| H | 5,19 | 5,20 |
| N | 18,81 | 18,62 |
| S | 7,17 | 7,43 |
| Cl | 7,93 | 7,66 |

b) 4,46 g Methylen (6R,7R)-7-amino-3-[(5-methyl-2H-tetrazol-2-yl)methyl]-8-oxo -5-thia-1-azabicyclo-[4.2.0]oct-2-en-2-carboxylat-pivalat Hydrochlorid werden in 50 ml Methylenchlorid gelöst. Man versetzt mit 4,79 g S-(2-Benzothiazolyl) 2-amino-$\alpha$-[(Z)-[1-(t-butoxycarbonyl-1-methyläthoxy]imino] -4-thiazol-thioacetat, rührt während 3 Stunden bei 20-25°, dampft die Lösung im Wasserstrahlvakuum bei 30° ein und löst den Rückstand in 50 ml Essigester. Unter Rühren versetzt man mit 200 ml Aether, wobei ein Harz ausfällt. Die überstehende flüssige Phase wird abdekantiert. Das Harz wird in 50 ml Essigester gelöst, worauf man unter Rühren auf 300 ml Aether giesst. Das amorph ausgefallene Material wird abgenutscht, mit Aether und tiefs. Petroläther gewaschen und über Nacht im Wasserstrahlvakuum bei 35° getrocknet. Man erhält 6,55 g (86,4%) Methylen (6R,7R)-7-[(Z)-2-(2-amino-4-thiazolyl) -2-[[1-(t-butoxycarbonyl)-1-methyläthoxy]imino]acetamido] -3-[(5-methyl-2H-tetrazol-2-yl)methyl]-8-oxo-5-thia -1-azabicyclo[4.2.0]oct-2-en-2-carboxylat-pivalat Hydrochlorid als beigen, amorphen Festkörper ($C_{29}H_{39}N_9O_9S_2$•HCl; MG: 758,266).

| Mikroanalyse: | | |
|---|---|---|
| | Ber. | Gef. |
| C | 45,94 | 45,73 |
| H | 5,32 | 5,56 |
| N | 16,63 | 16,37 |

Beispiel 6

4,46 g Methylen (6R,7R)-7-amino-3-[(5-methyl-2H-tetrazol-2-yl)methyl]-8-oxo -5-thia-1-azabicyclo[4.2.0]-oct-2-en-2-carboxylat-pivalat Hydrochlorid werden in 60 ml Methylenchlorid gelöst. Man versetzt mit 4,5 g S-(2-Benzothiazolyl)-2-amino-4-thiazol-thioglyoxylat (Z)-O-[(t-butoxycarbonyl)methyl]oxim und rührt 3 Stunden bei 20-25°. Die Lösung wird im Wasserstrahlvakuum bei 30° eingedampft. Der Rückstand wird in 50 ml Essigester gelöst. Es wird unter Rühren mit 200 ml Aether versetzt, vom amorph ausgefallenen Material abgenutscht und mit Aether gewaschen. Das erhaltene Material wird in 30 ml Essigester gelöst. Unter

Rühren wird auf 300 ml Aether gegossen. Das amorph ausgefallene Material wird abgenutscht, mit Aether und tiefs. Petroläther gewaschen und über Nacht im Wasserstrahlvakuum bei 40° getrocknet. Man erhält 6,2 g (84,9%) Methylen (6R,7R)-7-[(Z)-2-(2-amino-4-thiazolyl) -2-[[t-butoxycarbonyl)methoxy]imino]-acetamido]-3-[5-methyl -2H-tetrazol-2-yl)methyl]-8-oxo-5-thia-1-azabicyclo[4.2.0]oct-2-en-2-carboxylat-pivalat Hydrochlorid als beigen, amorphen Festkörper ($C_{27}H_{35}N_9O_9S_2 \cdot HCl$; MG: 730,212).

| Mikroanalyse: | | |
|---|---|---|
| | Ber. | Gef. |
| C | 44,41 | 44,32 |
| H | 4,97 | 5,18 |
| N | 17,26 | 17,00 |

Beispiel 7

26,1 g Methylen (6R,7R)-7-amino-3-[(5-methyl-2H-tetrazol-2-yl)methyl]-8-oxo -5-thia-1-azabicyclo[4.2.0]-oct-2-en-2-carboxylat-pivalat Hydrochlorid werden in 260 ml Methylenchlorid gelöst. Man versetzt mit 20,44 g S-(2-Benzothiazolyl)-2-amino-4-thiazol-thioglyoxylat (Z)-O-methyloxim, rührt die Lösung 2 Stunden bei 20-25° und dampft anschliessend im Wasserstrahlvakuum bei 30° ein. der Rückstand wird in 350 ml Aceton gelöst. Man lässt 1,5 Stunden unter Rühren kristallisieren, nutscht ab, wäscht mit Aceton und n-Hexan und trocknet über Nacht im Wasserstrahlvakuum bei 35°. Das erhaltene Material (33,15 g farblose Kristalle) werden in 100 ml Methanol gelöst, worauf man mit 100 ml Aethanol und anschliessend mit 130 ml n-Hexan versetzt, 30 Minuten bei 20-25° kristallisieren lässt, weitere 70 ml n-Hexan unter Rühren innerhalb von etwa 30 Minuten dazutropft, abnutscht und das erhaltene Material mit Aethanol/n-Hexan (1:1) und n-Hexan wäscht und über Nacht im Wasserstrahlvakuum bei 35° trocknet. Man erhält 29,5 g (80,16%) Methylen (6R,7R)-7-[(Z)-2-(2-Amino-4-thiazolyl) -2-(methoxyimino)acetamido]-3-[(5-methyl-2H-tetrazol -2-yl)methyl)-8-oxo-5-thial-1-azabicyclo[4.2.0]oct-2-en -2-carboxylat-pivalat Hydrochlorid als weisse Kristalle ($C_{22}H_{27}N_9O_7S_2 \cdot HCl$: MG: 630,095).

| Mikroanalyse: | | |
|---|---|---|
| | Ber. | Gef. |
| C | 41,94 | 42,02 |
| H | 4,48 | 4,40 |
| N | 20,01 | 20,14 |

Beispiel 8

3,25 g Methylen (2S,5R,6R)-6-amino-3,3-dimethyl-7-oxo-4-thia -1-azabicyclo[3.2.0]heptan-2-carboxylat-pivalat Hydrochlorid werden in 30 ml Methylenchlorid gelöst, worauf man mit 3,1 g S-(2-Benzothiazolyl)-2-amino-4-thiazol-thioglyoxylat (Z)-O-methyloxim versetzt, 2 Stunden bei 20-25° rührt und die erhaltene Lösung durch ein Faltenfilter filtriert. Das Filtrat wird unter Rühren auf 200 ml Isopropyläther gegossen. Das amorph ausgefallene Material wird abgenutscht, mit Isopropyläther gewaschen und in 250 ml n-Butylacetat gelöst. Im Wasserstrahlvakuum bei 30° wird dann auf ein Volumen von 100 ml eingeengt. Unter Rühren versetzt man mit 100 ml Aether, nutscht das ausgefallene Material ab, wäscht mit Aether und tiefs. Petroläther und trocknet über Nacht im Hochvakuum bei 40°. Man erhält 3,7 g (76%) Methylen (2S,5R,6R)-6-[(Z)-2-(2-amino-4-thiazolyl) -2-(methoxyimino)acetamido]-3,3-dimethyl-7-oxo-5-thia -1-azabicyclo[3.2.0]-heptan-2-carboxylat-pivalat Hydrochlorid als gelblich-amorphen Festkörper.

| Mikroanalyse: | | |
|---|---|---|
| | Ber. | Gef. ($H_2O$-frei) |
| C | 43,67 | 43,39 |
| H | 5,13 | 5,13 |
| N | 12,79 | 12,91 |

Beispiel 9

1,3 g Anilin-Hydrochlorid werden in 50 ml Methylenchlorid suspendiert. Man versetzt mit 3,5 g S-(2-Benzothiazolyl)-2-amino-4-thiazol-thioglyoxylat (Z)-O-methyloxim und rührt 1,5 Stunden bei 20-25°, wobei man eine Lösung erhält, aus welcher das gewünschte Produkt auskristallisiert. Man nutscht ab, wäscht mit Methylenchlorid und trocknet im Wasserstrahlvakuum bei 30°. Das erhaltene Material (3,1 g beige Kristalle) wird in 31 ml Methanol in der Wärme gelöst. Man kühlt auf 20° ab, versetzt mit 30 ml Aether, nutscht das auskristallisierte Material ab, wäscht mit Methanol/Aether (1:1) und Aether und trocknet über Nacht im Hochvakuum bei 40°. Man erhält 2,35 g (75,1%) 2-Amino-4-thiazolglyoxylanilid (Z)-O-methyloxim Hydrochlorid als beige Kristalle ($C_{12}H_{12}N_4O_2S \cdot HCl$; MG: 312,775).

| Mikroanalyse: | | |
|---|---|---|
| | Ber. | Gef. |
| C | 46,08 | 46,20 |
| H | 4.19 | 4,20 |
| N | 17,91 | 17,98 |

Beispiel 10

3,64 g 1-[[(6R,7R)-7-Amino-2-carboxy-8-oxo-5-thia -1-azabicyclo[4.2.0]oct-2-en -3-yl]methyl]-pyridiniumhydroxid-inneres Salz Dihydrochlorid werden in 250 ml Methanol gelöst. Nun gibt man 5,74 g S-(2-Benzothiazolyl) 2-amino-α-[(Z)-[1-(t-butoxycarbonyl)-1-methyläthoxy]imino] -4-thiazol-thioacetat und 150 ml Methylenchlorid dazu und rührt 2 Stunden bei 20-25°. Die erhaltene gelbe Lösung wird im Wasserstrahlvakuum bei 30° eingedampft, und der Rückstand wird mit 100 ml Aceton verrührt. Der Festkörper wird abgenutscht, mit Aceton gewaschen und im Vakuum bei 25° getrocknet. Das erhaltene Material (5,6 g beige-amorpher Festkörper) wird zwischen 50 ml Wasser und 50 ml Methylenchlorid verteilt. Die wässrige Phase wird abgetrennt, zweimal mit Methylenchlorid gewaschen und über Nacht im Hochvakuum lyophilisiert. Man erhält 4,2 g (62,2%) 1-[[(6R,7R)-7-[(Z)-(2-Amino-4-thiazolyl)-2-[[1 -(t-butoxycarbonyl)-1-methyläthoxy]imino]acetamido] -2-carboxy-8-oxo-5-thia-1-azabicyclo[4.2.0]oct-2-en -3-yl]methyl]-pyridiniumhydroxyd-inneres Salz Dihydrochlorid als beiges Lyophilisat ($C_{26}H_{30}N_6O_7S_2 \cdot 2HCl$; MG: 675,603).

| Mikroanalyse: | | |
|---|---|---|
| | Ber. | Gef. ($H_2O$-frei) |
| C | 46,22 | 46,29 |
| H | 4,77 | 4,68 |
| N | 12,44 | 12,51 |
| S | 9,49 | 9,43 |
| Cl | 10,50 | 10,48 |

Beispiel 11

3,09 g (6R,7R)-7-Amino-3-acetoxymethyl-8-oxo-5-thia -1-azabicyclo[4.2.0]oct-2-en-2-carbonsäure Hydrochlorid werden in 50 ml N,N'-Dimethylacetamid gelöst. Man versetzt mit 4,2 g S-(2-Benzothiazolyl)-2-amino-4-thiazol-thioglyoxylat (Z)-O-methyloxim, rührt die Lösung 30 Minuten bei 20-25° und dampft anschliessend im Hochvakuum bei 35° ein. Der Rückstand wird in 25 ml Aceton gelöst. Unter Rühren giesst man auf 200 ml Aether, filtriert das ausgefallene Material ab, wäscht es mit Aether und trocknet im Wasserstrahlvakuum bei 30°. Das erhaltene Material (6,5 g gelblich-amorpher Festkörper) wird in 100 ml Isopropanol unter Zusatz von 2 ml 5N-Salzsäure in Isopropanol 1 Stunde bei 25° gerührt, wobei Kristallisation eintritt. Die Kristalle werden abgenutscht, mit Isopropanol und tiefs. Petroläther gewaschen und über Nacht im Hochvakuum bei 30° getrocknet. Man erhält 3,55 g (72,15%) (6R,7R)-3-(Acetoxymethyl)-7-[2-(2-amino-4-thiazolyl) -2-(methoxyimino)acetamido]-8-oxo-5-thia -1-azabicyclo[4.2.0]oct-2-en-2-carbonsäure Hydrochlorid als beige Kristalle ($C_{16}H_{17}N_5O_7S_2$•HCl•0,3 Isopropanol; MG: 509,95).

| Mikroanalyse: | | |
|---|---|---|
| | Ber. | Gef. |
| C | 39,81 | 40,18 |
| H | 4,03 | 4,20 |
| N | 13,73 | 13,77 |

Beispiel 12

3,75 g (6R,7R)-7-Amino-3-methyl-8-oxo-5-thia-1-azabicyclo[4.2.0]oct -2-en-2-carbonsäure Hydrochlorid werden in 75 ml N,N'-Dimethylacetamid gelöst, worauf man mit 6,3 g S-(2-Benzothiazolyl)-2-amino-4-thiazol-thioglyoxylat (Z)-O-methyloxim versetzt, 30 Minuten bei 20-25° rührt und anschliessend im Hochvakuum bei 35° eindampft. Der Rückstand wird mit 500 ml Methylenchlorid verrührt. Das ausgefallene Material wird abgenutscht, mit Methylenchlorid und Aether gewaschen und im Wasserstrahlvakuum bei 25° getrocknet. Das erhaltene Material (6,3 g nahezu farbloser amorpher Festkörper) wird in 100 ml Isopropanol unter Zusatz von 3 ml 5N-Salzsäure in Isopropanol 2 Stunden bei 20-25° gerührt, wobei Kristallisation eintritt. Die Kristalle werden abgenutscht, mit Isopropanol und Aether gewaschen und in 40 ml Aethanol suspendiert. Die Suspension wird 30 Minuten bei 50° gerührt. Dann wird auf 20° abgekühlt, und der Festkörper wird abgenutscht, mit Aethanol und n-Hexan gewaschen und über Nacht im Hochvakuum bei 30° getrocknet. Man erhält 2,4 g (37%) (6R,7R)-7-[(Z)-2-(2-Amino-4-thiazolyl) -2-(methoxyimino)acetamido]-3-methyl-8-oxo-5-thia -1-azabicyclo[4.2.0]oct-2-en-2-carbonsäure Hydrochlorid als farblose Kristalle ($C_{14}H_{15}N_5O_5S_2$•HCl; MG: 433,885).

| Mikroanalyse | | |
|---|---|---|
| | Ber. | Gef. ($H_2O$-frei) |
| C | 38,76 | 39,16 |
| H | 3,72 | 3,71 |
| N | 16,14 | 16,22 |

Beispiel 13

2,23 g Methylen (6R,7R)-7-amino-3-[(5-methyl-2H-tetrazol-2-yl)methyl]-8-oxo -5-thia-1-azabicyclo[4.2.0]-oct-2-en-2-carboxylat-pivalat Hydrochlorid werden in 50 ml Methylenchlorid gelöst, worauf man mit 2,71 g (S)-(2-Benzothiazolyl)-thiobenzoat versetzt, 24 Stunden bei 20-25° rührt und die Lösung im Wasserstrahlvakuum bei 30° eindampft. Der Rückstand wird mit 25 ml Aether 1 Stunde verrührt. Man nutscht ab und wäscht mit Aether. Man löst das Material in 40 ml Methylenchlorid, gibt 80 ml Isopropyläther dazu und destilliert im Wasserstrahlvakuum bei 30° das Lösungsmittel ab, wobei das Produkt kristallisiert. Die Kristalle werden abgenutscht, mit Isopropyläther gewaschen und über Nacht im Hochvakuum bei 30° getrocknet. Man erhält 1,2 g (46,7%) Methylen (6R,7R)-7-benzamido-3-[(5-methyl-2H-tetrazol-2-yl)methyl] -8-oxo-5-thia-1-azabicyclo[4.2.0]oct-2-en-2-carboxylat-pivalat als weisse Kristalle ($C_{23}H_{26}N_6O_6S$; MG: 514,557).

| Mikroanalyse: | | |
|---|---|---|
| | Ber. | Gef. |
| C | 53,69 | 53.40 |
| H | 5,09 | 5,26 |
| N | 16,33 | 16,24 |

## Patentansprüche

1. Verfahren zur Acylierung von Aminen mit 2-Benzothiazolylthioestern von Carbonsäuren, dadurch gekennzeichnet, dass man das Amin in Form eines Säureadditionssalzes zur Umsetzung bringt.

2. Verfahren nach Anspruch 1, dadurch gekennzeichnet, dass das Amin ein 7-Aminocephalosporin- oder ein 6-Aminopenicillinderivat ist.

3. Verfahren nach Anspruch 2, dadurch gekennzeichnet, dass das Amin eine Verbindung der allgemeinen Formel

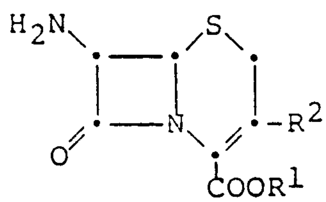

I

worin $R^1$ Wasserstoff oder eine für die Blockierung der 4-Carboxygruppe von Cephalosporinen übliche Gruppe und $R^2$ Wasserstoff oder eine für die 3-Stellung von Cephalosporinen übliche Gruppe bedeuten,
ist.

4. Verfahren nach Anspruch 3, dadurch gekennzeichnet, dass $R^1$ Wasserstoff oder die Gruppe -A-OCOR, A niederes Alkyliden, R niederes Alkyl oder niederes Alkoxy, $R^2$ Wasserstoff, Halogen, niederes Alkyl, niederes Alkenyl, niederes Alkanoyloxyalkyl, niederes Azidoalkyl, niederes Carbamoyloxyalkyl oder die Gruppe $-CH_2-S-Q$, $-CH=CH-S-Q$ oder $-CH_2-Q'$, Q eine über ein Kohlenstoffatom gebundene monocyclische 5- oder 6-gliedrige, partiell ungesättigte oder aromatische heterocyclische Gruppe und Q' eine über ein Stickstoffatom gebundene gesättigte, partiell ungesättigte oder aromatische heterocyclische Gruppe, welche bis zu 4 Stickstoffatome als Heteroatome enthält bedeuten und der Ausdruck "nieder" Reste mit bis zu 7 Kohlenstoffatome bedeutet.

5. Verfahren nach einem der Ansprüche 2-4, dadurch gekennzeichnet, dass der Benzothiazolylthioester eine Verbindung der allgemeinen Formel

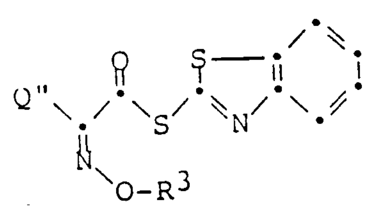

II

worin $R^3$ niederes Alkyl, niederes Alkenyl, niederes Alkanoyl oder die Gruppe -A'-COOR', A' niederes Alkylen, R' eine in der Cephalosporinchemie übliche Carboxyschutzgruppe und Q'' eine über ein Kohlenstoffatom gebundene, monocyclische 5- oder 6-gliedrige heteroaromatische Gruppe bedeuten und der Ausdruck "nieder" Reste mit bis zu 7 Kohlenstoffatome bedeutet, ist.

6. Verfahren nach Anspruch 5, dadurch gekennzeichnet, dass $R^3$ Methyl oder die Gruppe -A'-COOR', A' Methylen oder 2,2-Propylen, R' t-Butyl und Q'' 2-Amino-4-thiazolyl oder 2-Furanyl bedeuten.

7. Verfahren nach Anspruch 2, dadurch gekennzeichnet, dass man als Amin Methylen (6R,7R)-7-Amino-3-[(5-methyl-2H-tetrazol-2-yl)methyl]-8 -oxo-5-thia-1-azabicyclo[4.2.0]oct-2-en -2-carboxylat-pivalat und als Benzothiazolylthioester den (Z)-2-(2-Amino-4-thiazolyl)-2-(methoxyimino)essigsäure -2-benzothiazolylthioester verwendet.

8. Verfahren nach einem der Ansprüche 2-7, dadurch gekennzeichnet, dass man ein Säureadditionssalz mit einer aromatischen oder aliphatischen Sulfonsäure oder einer Mineralsäure verwendet.

9. Verfahren nach Anspruch 8, dadurch gekennzeichnet, dass man ein Säureadditionssalz mit p-Toluolsulfonsäure oder Salzsäure verwendet.

10. Verfahren nach einem der Ansprüche 1-9, dadurch gekennzeichnet, dass man als Lösungsmittel einen $C_1$-$C_7$ Alkohol, einen halogenierten $C_1$-$C_7$ Kohlenwasserstoff oder ein $C_1$-$C_7$ N,N-Dialkylfettsäureamid verwendet.

11. Verfahren nach einem der Ansprüche 1-10, dadurch gekennzeichnet, dass die Umsetzung in einem Bereich von 15°-30°C durchgeführt wird.

12. Anwendung der Verfahren nach einem der Ansprüche 1-11, zur Herstellung von antimikrobiell wirksamen 7-Acylamino-cephalosporinderivaten.

13. Anwendung der Verfahren nach einem der Ansprüche 1-11, zur Herstellung von Verbindungen der allgemeinen Formel

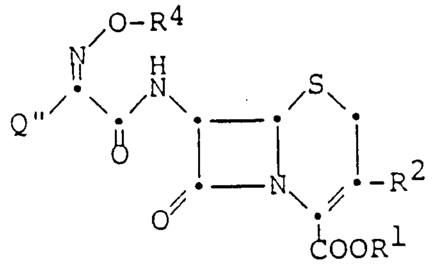

IV

worin $R^4$ Wasserstoff, niederes Alkyl, niederes Alkenyl oder die Gruppe -A'-COOH bedeutet, und $R^1$ und $R^2$ die in Anspruch 3 oder 4 und A' und Q'' die in Anspruch 5 oder 6 angegebene Bedeutung besitzen und der Ausdruck "nieder" Reste mit bis zu 7 Kohlenstoffatome bedeutet.

14. Anwendung der Verfahren nach einem der Ansprüche 1-11, zur Herstellung von Methylen (6R,7R)-7-[-(Z)-2-(2-amino-4-thiazolyl) -2-(methoxyimino)acetamido]-3-[(5-methyl-2H-tetrazol -2-yl)-methyl]-8-oxo-5-thia-1-azabicyclo[4.2.0]oct-2-en -2-carboxylat-pivalat.

## Claims

1. A process for the acylation of amines with 2-benzothiazolyl thioesters of carboxylic acids, characterized by reacting the amine in the form of an acid addition salt.

2. A process according to claim 1, characterized in that the amine is a 7-aminocephalosporin derivative or a 6-aminopencillin derivative.

3. A process according to claim 2, characterized in that the amine is a compound of the general formula

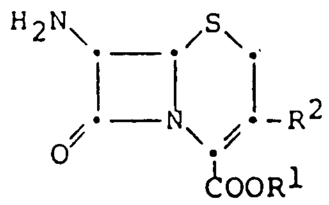

I

wherein $R^1$ signifies hydrogen or a group which is usual for the blocking of the 4-carboxy group of cephalosporins and $R^2$ signifies hydrogen or a group which is usual for the 3-position of cephalosporins.

**4.** A process according to claim 3, characterized in that $R^1$ signifies hydrogen or the group -A-OCOR, A signifies lower alkylidene, R signifies lower alkyl or lower alkoxy, $R^2$ signifies hydrogen, halogen, lower alkyl, lower alkenyl, lower alkanoyloxyalkyl, lower azidoalkyl, lower carbamoyloxyalkyl or the group $-CH_2$-S-Q, -CH=CH-S-Q or $-CH_2$-Q', Q signifies a monocyclic 5- or 6-membered, partially unsaturated or aromatic heterocyclic group bonded via a carbon atom and Q' signifies a saturated, partially unsaturated or aromatic heterocyclic group which contains up to 4 nitrogen atoms as the hetero atom(s) and which is bonded via a nitrogen atom, and the term "lower" denotes residues with up to 7 carbon atoms.

**5.** A process according to any one of claims 2-4, characterized in that the benzothiazolyl thioester is a compound of the general formula

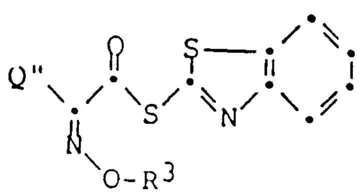

II

wherein $R^3$ signifies lower alkyl, lower alkenyl, lower alkanoyl or the group -A'-COOR', A' signifies lower alkylene, R' signifies a carboxy group which is usual in cephalosporin chemistry and Q" signifies a monocyclic 5- or 6-membered heteroaromatic group bonded via a carbon atom, and the term "lower" denotes residues with up to 7 carbon atoms.

**6.** A process according to claim 5, characterized in that $R^3$ signifies methyl or the group -A'-COOR', A' signifies methylene or 2,2-propylene, R' signifies t-butyl and Q" signifies 2-amino-4-thiazolyl or 2-furanyl.

**7.** A process according to claim 2, characterized in that methylene (6R,7R)-7-amino-3-[(5-methyl-2H-tetrazol-2-yl)methyl]-8-oxo-5-thia-1-azabicyclo[4.2.0]oct-2-ene-2-carboxylatepivalate is used as the amine and (Z)-2-(2-amino-4-thiazolyl)-2-(methoxyimino)acetic acid 2-benzothiazolyl thioester is used as the benzothiazolyl thioester.

**8.** A process according to any one of claims 2-7, characterized in that an acid addition salt with an aromatic aliphatic or sulphonic acid or a mineral acid is used.

**9.** A process according to claim 8, characterized in that an acid addition salt with p-toluenesulphonic acid or hydrochloric acid is used.

**10.** A process according to any one of claims 1-9, characterized in that a $C_1$-$C_7$ alcohol, a halogenated $C_1$-$C_7$ hydrocarbon or a $C_1$-$C_7$ N,N-dialkyl fatty acid amide is used as the solvent.

**11.** A process according to any one of claims 1-10, characterized in that the reaction is carried out in a temperature range of 15°-30°C.

**12.** The use of the process according to any one of claims 1-11 for the manufacture of antimicrobially-active 7-acylamino-cephalosporin derivatives.

**13.** The use of the process according to any one of claims 1-11 for the manufacture of compounds of the general formula

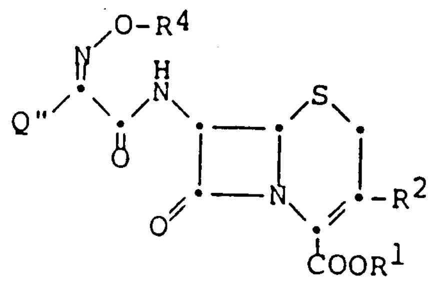

IV

wherein $R^4$ signifies hydrogen, lower alkyl, lower alkenyl or the group -A'-COOH, $R^1$ and $R^2$ have the significance given in claim 3 or 4 and A' and Q'' have the significance given in claim 5 or 6, and the term "lower" denotes residues with up to 7 carbon atoms.

**14.** The use of the process according to any one of claims 1-11 for the manufacture of methylene (6R,7R)-7-[(Z)-2-(2-amino-4-thiazolyl)-2-(methoxyimino)acetamido]-3-[(5-methyl-2H-tetrazol-2-yl)-methyl]-8-oxo-5-thia-1-azabicyclo[4.2.0]oct-2-ene-2-carboxylate pivalate.

**Revendications**

**1.** Procédé pour acyler des amines à l'aide de thioesters 2-benzothiazolyliques d'acides carboxyliques, caractérisé en ce que l'on fait réagir l'amine à l'état de sel formé par addition avec un acide.

**2.** Procédé selon la revendication 1, caractérisé en ce que l'amine est un dérivé de la 7-aminocéphalosporine ou de la 6-aminopénicilline.

**3.** Procédé selon la revendication 2, caractérisé en ce que l'amine est un composé de formule générale

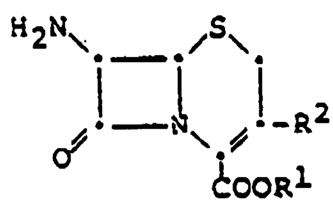

I

dans laquelle $R^1$ représente l'hydrogène ou un groupe utilisé couramment pour le blocage du groupe 4-carboxy des céphalosporines et $R^2$ représente l'hydrogène ou un groupe usuel en position 3 des céphalosporines.

**4.** Procédé selon la revendication 3, caractérisé en ce que $R^1$ représente l'hydrogène ou le groupe -A-OCOR dans lequel A est un groupe alkylidène inférieur, R un groupe alkyle inférieur ou alcoxy inférieur, $R^2$ représente l'hydrogène, un halogène, un groupe alkyle inférieur, alcényle inférieur, alcanoyloxyalkyle inférieur, azidoalkyle inférieur, carbamoyloxyalkyle inférieur ou le groupe $-CH_2-S-Q$, $-CH=CH-S-Q$ ou $-CH_2-Q'$, Q est un groupe hétérocyclique monocyclique à 5 ou 6 chaînons, partiellement insaturé ou aromatique, relié par l'intermédiaire d'un atome de carbone et Q' un groupe hétérocyclique saturé, partiellement insaturé ou aromatique, relié par l'intermédiaire d'un atome d'azote et qui peut contenir jusqu'à 4 atomes d'azote en tant qu'hétéroatomes, et l'expression "inférieur" s'applique à des groupes contenant jusqu'à 7 atomes de carbone.

5. Procédé selon l'une des revendications 2 à 4, caractérisé en ce que le thioester benzothiazolylique est un composé de formule générale

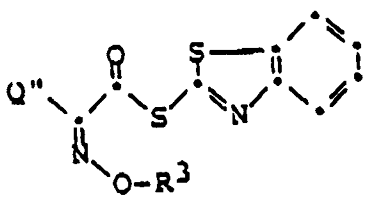

II

dans laquelle $R^3$ représente un groupe alkyle inférieur, alcényle inférieur, alcanoyle inférieur ou le groupe -A'-COOR', A' représente un groupe alkylène inférieur, R' un groupe protecteur usuel dans la chimie des céphalosporines pour le groupe carboxy et Q" un groupe monocyclique hétéroaromatique à 5 ou 6 chaînons relié par l'intermédiaire d'un atome de carbone, et l'expression "inférieur" s'applique à des groupes contenant jusqu'à 7 atomes de carbone.

6. Procédé selon la revendication 5, caractérisé en ce que $R^3$ représente le groupe méthyle ou le groupe -A'-COOR', A' un groupe méthylène ou 2,2-propylène, R' un groupe tert-butyle et Q" un groupe 2-amino-4-thiazolyle ou 2-furannyle.

7. Procédé selon la revendication 2, caractérisé en ce que l'amine utilisée est le méthylène (6R,7R)-7-amino-3-[(5-méthyl-2H-tétrazole-2-yl)-méthyl]-8-oxo-5-thia-1-azabicyclo[4.2.0]octa-2-ène-2-carboxylate-pivalate et le thioester benzothiazolylique est le thioester 2-benzothiazolylique de l'acide (Z)-2-(2-amino-4-thiazolyl)-2-(méthoxyimino)-acétique.

8. Procédé selon l'une dès revendications 2 à 7, caractérisé en ce que l'on utilise en tant que sel formé par addition avec un acide un sel formé par addition avec un acide sulfonique aliphatique ou aromatique ou avec un acide minéral.

9. Procédé selon la revendication 8, caractérisé en ce que l'on utilise en tant que sel formé par addition avec un acide un sel formé par addition avec l'acide p-toluène-sulfonique ou avec l'acide chlorhydrique.

10. Procédé selon l'une des revendications 1 à 9, caractérisé en ce que l'on utilise en tant que solvant un alcool en C1-C7, un hydrocarbure halogéné en C1-C7 ou un N,N-dialkylamide d'acide gras en C1-C7.

11. Procédé selon l'une des revendications 1 à 10, caractérisé en ce que l'on effectue la réaction dans l'intervalle de 15 à 30 ° C.

12. Application des procédés selon l'une des revendications 1 à 11 à la préparation de dérivés de 7-acylaminocéphalosporine possédant une activité antimicrobienne.

13. Application des procédés selon l'une des revendications 1 à 11 à la préparation de composés de formule générale

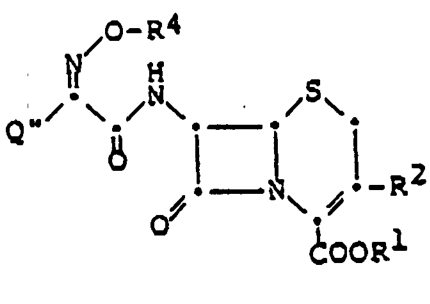

IV

dans laquelle $R^4$ représente l'hydrogène, un groupe alkyle inférieur, alcényle inférieur ou le groupe -A'-

COOH, et $R^1$ et $R^2$ les significations indiquées dans la revendication 3 ou 4 et A' et Q'' les significations indiquées dans la revendication 5 ou 6, et l'expression "inférieur" s'applique à des groupes contenant jusqu'à 7 atomes de carbone.

**14.** Application des procédés selon l'une des revendications 1 à 11 à la préparation du méthylène (6R,7R)-7-[(Z)-2-(2-amino-4-thiazolyl)-2-(méthoxyimino)acétamido]-3-[(5-méthyl-2H-tétrazole-2-yl)-méthyl]-8-oxo-5-thia-1-azabicyclo[4.2.0]octa-2-ène-2-carboxylate-pivalate.